# EUROPEAN PATENT SPECIFICATION

(11) **EP 1 871 288 B1**
(45) Date of publication and mention of the grant of the patent: **24.02.2016**
(21) Application number: 05756151.6
(22) Date of filing: 01.06.2005
(51) Int. Cl.: A61F 2/06, C23C 14/00

(54) **SELF-EXPANDABLE AND COLLAPSIBLE THREE-DIMENSIONAL DEVICES AND METHODS**
AUTOMATISCH AUFWEITBARE UND ZUSAMMENLEGBARE DREIDIMENSIONALE VORRICHTUNGEN UND VERFAHREN
DISPOSITIFS TRIDIMENSIONNELS AUTO-DEPLOYABLES ET ESCAMOTABLES ET PROCEDES DE FABRICATION

(30) Priority: 08.06.2004 US 577774 P
(43) Date of publication of application: 02.01.2008
(73) Proprietor: TINI ALLOY COMPANY, Berkeley, CA 94708-1951 (US)
(72) Inventor: GUPTA, Vikas, San Leandro, California 94577 (US); JOHNSON, David A., San Leandro, California 94577 (US); MENCHACA, Leticia, Berkeley, California 94702 (US); MARTYNOV, Valery, San Francisco, California 94121 (US)
(74) Representative: Price, Nigel John King
(86) International application number: PCT/US2005/019078
(87) International publication number: WO 2005/122714

(56) References cited:
- EP-A1- 1 779 817
- EP-A2- 0 836 839
- WO-A1-98/42277
- US-B2- 6 533 905

## Description

### Cross -Reference to Prior Application

This application claims the benefit under 35 USC §119(e) of United States provisional application serial no. 60/577,774 filed June 8, 2004.

### Background of the Invention

### 1. Field of the Invention

This invention relates to three-dimensional thin film devices and methods of fabricating the same, and more particularly to such devices and fabrication methods in the medical field that are adaptable for applications such as implantable endoluminal stents and stent-coverings and occlusion devices for neurovascular applications, as well as three-dimension thin film shapes, such as tubular, which can be expanded and collapsed in radial directions.

### 2. Description of the Related Art

In recent years many medical devices have been introduced that incorporate shape memory and superelastic materials, especially titanium nickel alloys, often referred to as TiNi or Nitinol. Two principal prior art products are guidewires for catheters and stents used in the treatment of vascular disease. Use of Nitinol in stents fabricated from small diameter Nitinol tubing has grown rapidly. However, stents made from tubing are generally lacking in the degree of flexibility required to treat small blood vessels in the brain that must be accessed intralumenally through tortuous distal paths in the carotid artery.

Accordingly there is a need for improved devices to be used in the intracranial vasculature. Thin film TiNi has desirable characteristics for fabricating these devices, especially because it can be rolled, folded, or otherwise compressed for insertion through micro-catheters.

Production of complete systems for minimally invasive vascular treatment involves joining of components by welding, brazing, soldering, and adhesives. Superior performance can be achieved if the number of such attachments is minimized. Additionally, welding of thin film (micrometers thick) presents novel problems. It is desirable to make the device all in one piece to achieve maximum flexibility, strength, and minimal thrombo-genicity.

The most common method of producing thin metal films is by vacuum sputtering, generally onto a planar substrate. Sputtering onto three-dimensional substrates can be accomplished by rotation of the substrate in or near the plasma, and by cylindrical sputtering. While it has been shown that it is possible to sputter three-dimensional shapes, it is also known that the material thus produced by this method is not of the highest quality, and the methods do not lend themselves to production of large numbers of devices at low cost.

In three-dimensional deposition it is difficult to achieve good crystal structure of the deposited thin film alloy. This requires shielding to produce line-of-sight normal deposition, otherwise columnar structure appears with poor intra-crystalline and inter-crystalline adhesion. Brittleness results.

It is also difficult to remove the three-dimensional structure from the substrate. Etchants must be extremely selective, and must not interact with the thin film material, generally TiNi or TiNi-based.

Miniature devices made of free-standing thin film shape memory alloys such as Nitinol have potential applications in medicine, particularly in minimally invasive surgery of the vasculature. For a majority of endolumenal applications it is essential that these devices have three-dimensional shapes, i.e. cones, cylinders, and hemispheres, to take advantage of superelastic and shape memory properties. In applications relating to tissue engineering, these three-dimensional shapes of Nitinol thin film can be used as a base structural material or scaffold on which to grow artificial tissue cells. For example, tissue grown on a thin film cylinder will produce an artificial blood vessel in a tubular shape.

The need has therefore been recognized for thin film devices that obviate the foregoing and other limitations and disadvantages of prior art devices of the type described. Despite the various devices of the type in the prior art, there has heretofore not been provided a suitable and attractive solution to these problems.

WO-A1-98/42277, on which the precharacterizing portion of the independent claims are based, discloses on expandable tubular stent formed from metal and which comprises columns of expansion strut pairs joined by plural connecting struts.

A general object of this invention is to provide new and improved three- dimensional thin film devices and methods of fabricating the same.

A further object is to provide devices and methods of the type described which are self-expandable and self-collapsible.

A further object is to provide devices and methods of the type described which are adaptable for -use as implantable endoluminal stents and stent-coverings and occlusion devices for neurovascular applications, as well as three-dimension thin film shapes, such as tubular, which can be expanded and collapsed in radial -directions.

The invention provides a method as defined in claims 1 to 3 and a corresponding device as defined in claims 4 to 7. Where in the following the word invention is used and/or features are presented as optional this should be interpreted in such way that protection is sought for the invention as claimed.

The self-expandable and self- collapsible properties enable use of the devices in medical applications such as implantable endoluminal stents and stent-coverings as well as occlusion devices for neurovascular applications. Further uses include devices of three-dimension thin film shapes, such as tubular, which can be expanded and collapsed in radial directions.

### Brief Description of The Drawings

Fig. 1 is a flow chart showing the method steps of fabricating devices in accordance with the invention.
Fig. 2 is a side view of a thin film expandable stent device fabricated by the method of Fig. 1 and shown in its collapsed state.
Fig. 3 is an enlarged view of a portion of the device shown in Fig. 2.
Fig. 4 is a side view of the device fabricated by the method of Fig. 2 shown in its expanded state.
Fig. 5 is an enlarged view of a portion of the device shown in Fig. 4.
Fig. 6 is a fragmentary view of a stent device of the invention shown placed around an inner bulkier stent.

### Description Of The Preferred Embodiments

### Method Of Fabricating Devices

Deploying or implanting medical devices using micro-catheters through tortuous small blood vessels, in the brain for example, requires the devices to be extremely flexible and miniaturized. Such flexible and miniaturized devices can be made using extremely thin films (usually about 2 to 50 microns thick). Thin film deposition techniques are used to fabricate such devices. Although, thin film can be fabricated using several deposition techniques, sputter deposition techniques are mainly used for fabricating thin film stents. All thin film sputtering methods comprise three basic steps: 1) generation of the atomic or ionic species from the metal or alloy material target, 2) transport of these species from the target to a substrate through a gas or a plasma medium, and 3) condensation of these species on the substrate surface to form a solid thin film. In the context of this invention, the target can be of any metal or metal alloys, the substrate can be a polished silicon or glass wafer and argon gas can be used to generate intense plasma.

To form the devices, thin film is sputtered in a process chamber which is kept in a very high vacuum and contains the target material and a table to mount the substrates. In the context of the invention, since the devices are fabricated using a multi-layered thin film system, the process chamber is capable of accommodating multiple sputtering targets. The chamber allows sputtering from one or all the targets in both RF and DC sputtering mode. In the process chamber, vacuum in the range of low 10⁻⁷ torr is accomplished by the help of one or more vacuum pumps such as mechanical pump, cryo pump and turbo molecular pump. Argon gas (or other inert gas) at a low pressure (around few millitorr) is introduced into the chamber. Upon applying a high voltage to the target material from a DC or an RF power supply, a glow discharge is created which dissociates the argon atoms into intense cloud of ions called plasma. These high energy ions in the plasma are accelerated to the target material and are capable of dislodging atoms from the target surface. These dislodged atoms from the target fall on the substrate surface which is mounted on the table thereby depositing thin film on the substrate.

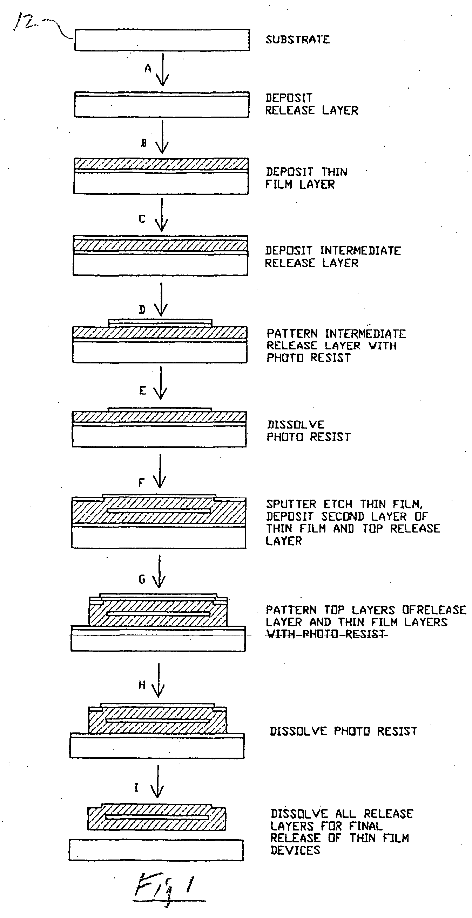

The flow chart of Fig. 1 shows the general method steps to fabricate three-dimensional shapes of thin films. Multiple layers of thin film device material and sacrificial material are sputter deposited sequentially on a surface of substrate 12 in a process chamber (not shown). The device material is of a shape memory alloy (also called SMA) such as TiNi (also called nitinol), or other derivative alloys of nitinol, stainless steel, or the like. Nitinol can be sputtered from one TiNi target or can be sputtered from two separate Ti and Ni targets. TiNi thin film of different composition can also be obtained by co-sputtering TiNi with another Ti or a Ni target together. Sacrificial material can be chromium, aluminum, copper, TiCuSil or any other metal or alloy. To accomplish such multiple layers, the process chamber is equipped with two separate targets - one for the thin film device layer and the other for the sacrificial layer. Although these layers can be sputter deposited in several ways, in most cases, the device layer is deposited using DC sputtering and the sacrificial layer using RF sputtering. Sputtered thin film alloy can also be achieved by either sputtering from a single alloy target or by co-sputtering from multiple targets. Polished and oxidized silicon wafers are used as substrates and are loaded into the chamber which is pumped down to achieve vacuum in a low 10⁻⁷ torr range. Highly polished and flat glass wafer can also be used as a substrate.

As shown in Fig. 1, a thin sacrificial layer is sputter deposited on the oxidized silicon wafer or substrate 12 using RF sputtering at argon pressure of about 2 millitorr (Fig. 1, step A). Thickness of deposited thin film can be 500Å or more. A thin layer of device material is sputter deposited on top of the sacrificial layer using DC sputtering at an argon pressure of about 2 millitorr (Fig. 1, step B). Thickness of the deposited device layer can be from 1 microns to 40 microns. A thin sacrificial layer is then sputter deposited on top of the device layer (Fig. 1, step C). Typical thickness of this layer is about 1000Å. This layer plays two roles: 1) it acts as a protective layer for the underlying device layer during subsequent lithography steps, and 2) it acts as a sacrificial layer which in the final steps of fabrication is dissolved away chemically in order to selectively create a pocket between the two device layers.

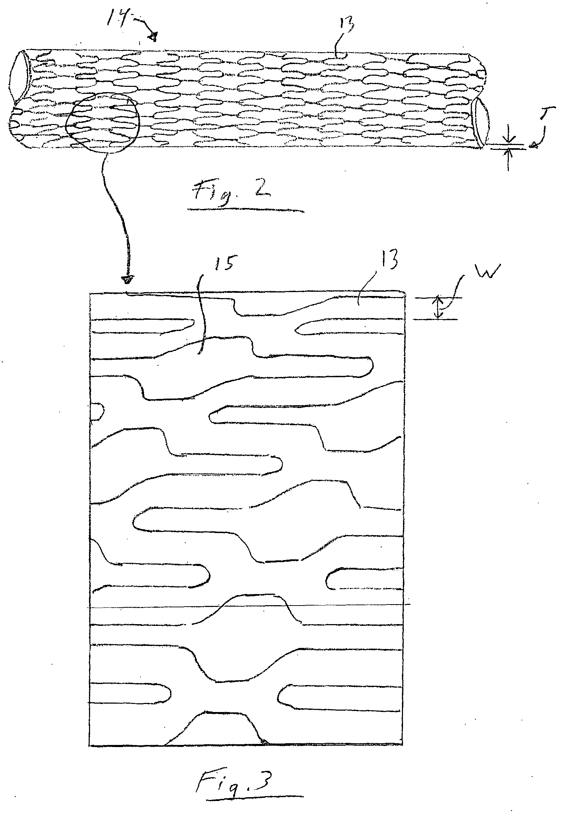

To make three-dimensional thin film shapes as shown in Figs. 2 to 5, two photo masks (mask1 and mask2) with appropriate pattern designs are required. A photo mask is typically a high quality chrome coated glass plate with desired device patterns etched on to the chrome layer which finally results in opaque and clear regions correspondingly on the mask plate. Designs on the mask1 determine the final three-dimentionality of the device - for example, solid triangular designs for cones, rectangular for cylinders, semicircular for hemispheres, etc. Mask2 contains designs for final definition of the device, fenestration patterns or any other surface pattern which may be needed for the final device. Mask1 is used to pattern the top sacrificial layer deposited on the wafer described above.

To pattern the above sputtered wafer with designs on mask1, typical steps of micro-photolithography technique are followed. A thin layer of positive photo resist liquid is spin-coated on the above wafer at about 4000 rpm and baked at 90°C in a clean room convection oven. Using an ultraviolet light mask aligner, the wafer and mask1 are aligned and the photo resist layer is UV exposed though the mask plate which transfers the patterns from mask1 on to the photo resist layer. The wafer with exposed photo resist is immersed in developer solution to selectively remove the exposed sections of the photo resist thus creating windows in the photo resist layer on the wafer. When immersed in a chemical etchant, these windows in the photo resist allow for the selective etching of the sacrificial layer as shown in Fig. 1, step D. After patterning the sacrificial layer, the photo resist layer is chemically dissolved away by immersing in a solvent (Fig. 1, step E) which also concludes the photolithography process.

The wafer with the selectively etched sacrificial layer is loaded back into the sputtering chamber which is pumped down to low 10⁻⁷ torr vacuum. A second layer of device material and a sacrificial layer are sputter deposited on the substrate (Fig. 1, step F). Thickness of these layers may remain same as in steps B and C.

In case of sputtered device material being nitinol or any other shape memory alloy, the material is heat-treated at 500°C in vacuum for crystallization so that the material exhibits the properties of shape memory alloy and superelasticity. To achieve this, the sputtered wafer from above is mounted on a hot plate which is situated in a vacuum chamber. In a reasonable vacuum, the power to the hot plate is turned on and the temperature is monitored at the bottom and top end of the wafer using thermocouples.

As illustrated in Fig. 1, step G, the photo resist is spun coated again to pattern the layers with designs in mask2 using the photolithography steps described above. In this step, after etching the top sacrificial layer, the underlying device layers are also chemically etched with the same mask shape in order to define the device's outer features. This is followed by the complete removal of the photo resist layer (Fig. 1, step H). To separate the devices from the surface of the substrate, the whole wafer with patterned layers is immersed into the chemical etchant to completely dissolve the sacrificial layer (Fig. 1, step I). The etchant for this purpose should be such that it should etch the sacrificial material selective to the device layer. This etching not only separates the devices from the substrate surface but also selectively creates an empty pocket between the two device layers by etching away the sacrificial layer from between.

Fenestration patterns can also be added in the device layer. To create fenestration patterns, the photo resist layer in Fig. 1, step G is patterned using mask2 which contains the necessary fenestration patterns. It is clear that the basic process sequence to fabricate thin film devices with fenestration patterns is same as the one described above except for the added designs on mask2. As described above, after patterning the photo resist layer with fenestration patterns, sacrificial and device layers are patterned by chemical etching (Fig. 1, step G).

### Self-expandable and Self-collapsible Nitinol Superelastic stents

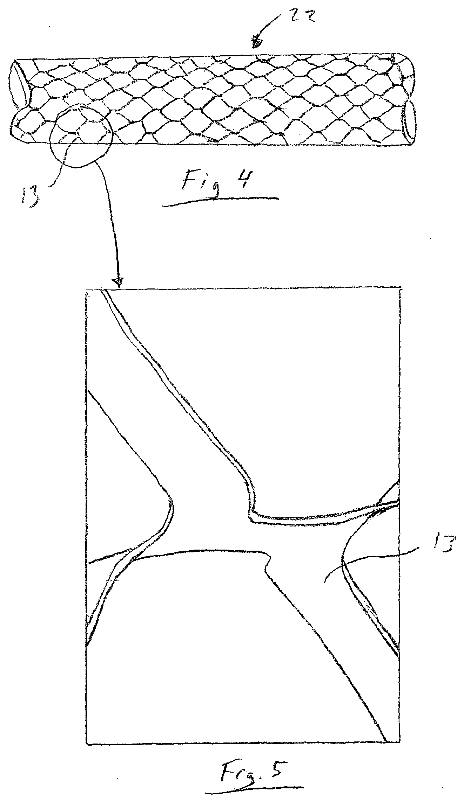

To make TiNi a thin film tubular structure 14 that can be self-expandable or self-collapsible, the multi-layered thin film devices are patterned with a device shape as shown in Fig. 2. Though there are many variations to this shape that will allow the self-expansion or self-collapsible of the thin film tubular device, the principle features include long TiNi thin film struts 13 with long narrow openings 15 between the struts and struts connected at the end of the long openings. The long openings between the struts provide the expandability in radial direction when these are stretched from long narrow opening shape to an open diamond shape. The length of the narrow opening determines the amount of expansion of shape. Fig. 4 illustrates a TiNi thin film tubular device 22 after being radially expanded. In this invention, the uniqueness comes from the way that the struts are bent and stretched at the apex when stretched radially. As shown by the partially enlarged view of device 22 in Fig. 5 the bending of struts 13 at various apexes is fairly complex but very uniform. The magnified image from of Fig. 5 shows the complex bending of struts in multiple directions after the thin film tubular device has been radially expanded. This unique method of bending provides a significant amount of expandability without causing any plastic deformation to the TiNi thin film struts. The partially enlarged view of the device shape at Fig. 3 illustrates a ratio of film thickness T (Fig. 2) to strut width W (Fig. 3) in the range of 1:5 to 1:9, and preferably 1:7. This low T:W ratio allows a high degree of expandability. Typically, for any other form of material, a 1:1 ratio between strut thickness to strut width is required to obtain a significant amount of expandability. We also believe that the features such as the bending of struts in different planes may be helpful in getting a better anchoring of the stent in the blood vessel.

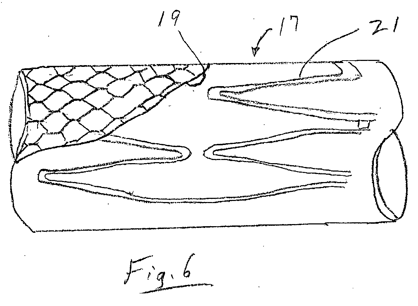

This device shape can be used in making either a self-expandable or a self-collapsible thin film tubular device. In case of a self-expandable device, the thin film superelastic device with the shape shown in Fig. 2 is expanded to final shape (using a mandrel) and then is heated in vacuum to 500°C for shape-setting at that final shape. The shape-set device is then cooled in the vacuum and removed from the mandrel. After shape-setting, a load can be applied to the device to collapse radially to a smaller diameter (for the purpose of loading into the catheter). When the load is released, the tubular device bounces back to its original dimensions at which it was shape-set. Fig. 6 shows a self expandable thin film superelastic tubular device. A self-expandable nitinol thin film superelastic tubular device can be used as a stent. A self-expandable stent is collapsed before loading into a catheter and inserted into the blood vessel. Inside the blood vessel, when the stent is pushed out of the catheter, it returns to its original larger diameter shape. A self-expandable stent that is made from a sputtered TiNi film material should have TiNi wall thickness of 5 to 50 microns in order to achieve sufficient radial force from the stent. A self-expandable thin film stent can be deployed in blood vessel as an occlusion device for the treatment of an aneurysm or as a clot filtering device.

In case of a self-collapsible device, the thin film superelastic tubular device with the shape, as shown in Fig. 2, is heated for shape-set in vacuum to 500°C with no radial expansion. The shape-set device is cooled in vacuum and removed from mandrel. After shape-setting, the device can be mounted onto a larger diameter mandrel and expanded radially to that diameter without causing any plastic deformation in the thin film material. When the mandrel is removed, the tubular device collapses back to its original diameter at which it was shape-set. A self-collapsible thin film superelastic stent can be used as a stent-cover. A thin film stent cover can be placed on an expanded stronger stent and then can be collapsed to a smaller diameter in order to load them inside a catheter. Fig. 6 shows a thin film stent cover that has been placed on top of an inner stent made from a bulk nitinol material. A self collapsible thin film stent will collapse in a very controlled manner along with the inner stent as the inner stent is forced to collapse. When the thin film covered stent is pushed out of the catheter, the inner stent, being stronger than thin film stent, automatically expands forcing the thin film stent-cover to expand at the same time for the final deployment in the blood vessel.

Fig. 2 shows a thin film expandable stent 14, fabricated by the method of Fig. 1, in a collapsed state. The enlarged view in Fig. 3 of collapsed stent 14 exhibits no bending of its struts 16.

Fig. 4 shows stent 14 after being expanded. The enlarged view in Fig. 5 of expanded stent 14 shows the complex network of bending of struts at different planes; there is no permanent deformation in the SMA material at the bending positions.

### Attachment of thin film stent-cover to an inner stent

The invention also comprises device shape 17 of Fig. 6 that enables attachment of a thin film stent cover 19 about an inner bulky stent 21. The device shapes for attachment are made on the thin film stent covers. Using the above described multiple layer method of making three-dimensional structures, separate tubular structures within each layer are fabricated at the two ends of the stent cover. These separate tubular structures are patterned so that they can be self-collapsible. In order for this attachment mechanism to work, the inner stent is fabricated with some struts with extra length protruding at the ends. Often stents come with such features because radio-opaque markers can be installed onto them. During the placement of the thin film stent cover on to a inner stent, these protruding struts can be inserted through those separate self-collapsible tubular structures on the thin film stent cover. Fig. 6 shows an attachment of the thin film stent cover 18 onto an inner bulkier stent 20.
thin film stent shown as a stent cover and placed on top of stent.

### Device shapes to improve flexibility of the stent

The invention also encompasses advanced stent shapes which not only expand or collapse in radial direction but also stretch and compress in an axial direction. In the conditions where a stent is inserted through tortuous bends in the blood vessel, the stent must be flexible enough to conform with the bending of the blood vessel.

The following comprise various applications for devices of the invention:
- medical device application of implantable endoluminal devices such as stents or stent-coverings made from superelastic thin film material. Particularly suitable in the applications where thin film stent-cover is placed on top of a thicker and stronger stent device for improved covering.
- the medical application of implantable endoluminal devices such as stand-alone self-expandable stents made from sputtered superelastic material. Particularly, implantable stent for neurovascular application in which a self expandable stent made from sputtered shape memory alloy thin film can be implanted as an occlusion device
- tubular shapes of shape memory alloy thin film with shapes that can be expanded and collapsed in radial direction.
- tubular shapes of shape memory alloy thin film with shapes that expands in the axial direction to allow bending.
- tubular shapes of shape memory alloy thin film with shapes that can be used for attaching the thin film stent-covers with the underlying thicker stent structure as a means to securing the two structures together without using any crimping, welding or suturing.
- device shapes enabling a significant radial expansion of the thin film tubular structures that are the results of a complex but very uniform bending of the thin film structures in multiple dimensions. Some portions of the thin film bend out-of-plane and some stay on-plane. When load is released, the self-collapsible structure returns to its original size with no presence of any bending. This complex bending of a fully expanded thin film stent-cover improves anchoring of stent in the blood vessel by providing extra friction.

## Claims

1. A method of fabricating a self-expandable three-dimensional device (14) comprising the steps of forming a device layer of a material, and shaping the device layer into a pattern comprising a plurality of struts (13) each of which have their opposite ends interconnected with opposite ends of adjacent struts (13), and the struts (13) having middle portions between their opposite ends with openings (15) between the middle portions of adjacent struts (13), the pattern further being shaped so that the openings (15) have a length longitudinally of one axis of the device (14) and a width orthogonal with the one axis with the length being longer that the width by an amount which is sufficient to enable the struts (13) to expand in a radial direction orthogonal to the one axis when the struts (13) are stretched from a long narrow opening shape to an open diamond shape; **characterised in that**: the device layer of a material is a thin film device layer comprising a shape memory alloy such that the device (14) is a thin film device, and **in that** when the struts are stretched radially of the device (14) some struts (13) bend out-of-plane and some stay on-plane.

2. A method as in claim 1 in which the layer is in the shape of a tube.

3. A method as in claim 1 in which the layer has a given thickness T and the struts each have a given width W with the ratio T: W being in the range of 1: 5 to 1:9.

4. A self-expandable three-dimensional device (14), the device having a pattern comprising a plurality of struts (13) each of which have their opposite ends interconnected with opposite ends of adjacent struts (13), and the struts (13) having middle portions between their opposite ends with openings (15) between the middle portions of adjacent struts (13), the pattern further comprising the openings (15) which have a length longitudinally of one axis of the device (14) and a width orthogonal with the one axis with the length being longer that the width by an amount which is sufficient to enable the struts (13) to expand in a radial direction orthogonal to the one axis when the struts (13) are stretched from a long narrow opening shape to an open diamond shape **characterised in that**: the device (14) is a thin film device (14) comprising a thin film device layer of a shape memory alloy, and **in that** some of the struts (13) bend out-of-plane and some stay on-plane when stretched radially.

5. A device (14) as in claim 4 in which the layer is in the shape of a tube.

6. A device (14) as in claim 4 in which the layer has a given thickness T and the struts (13) each have a given width W with the ratio T: W being in the range of 1: 5 to 1:9.

7. A device (14) as in claim 4 and futher comprising an inner stent (21), and the layer is in the shape of a hollow tube (19) which encloses about the outside of the inner stent (21).

## Patentansprüche

1. Verfahren zum Fertigen einer selbst ausdehnbaren dreidimensionalen Vorrichtung (14) umfassend die Schritte eines Ausbildens einer Vorrichtungsschicht eines Materials und Formen der Vorrichtungsschicht in ein Muster, welches mehrere Streben (13) umfasst, wobei deren gegenüberliegenden Enden jeweils mit gegenüberliegenden Enden von benachbarten Streben (13) verbunden werden, und wobei die Streben (13) Mittelabschnitte zwischen ihren gegenüberliegenden Enden mit Öffnungen (15) zwischen den Mittelabschnitten von benachbarten Streben (13) aufweisen, wobei das Muster darüber hinaus geformt wird, so dass Öffnungen (15) eine Länge längs einer Achse der Vorrichtung (14) und eine Breite orthogonal zu der Achse aufweisen, wobei die Länge einen Betrag länger als die Breite ist, welcher ausreicht, damit sich die Streben (13) in einer radialen Richtung orthogonal zu der Achse ausdehnen können, wenn die Streben (13) von einer langen schmalen Öffnungsform zu einer offenen Rautenform gedehnt werden; **dadurch gekennzeichnet, dass** die Vorrichtungsschicht eines Materials eine dünnschichtige Vorrichtungsschicht, welche eine Formgedächtnis-Legierung umfasst, ist, so dass die Vorrichtung (14) eine dünnschichtige Vorrichtung ist, und dass sich einige Streben (13) aus der Ebene biegen und einige auf der Ebene verbleiben, wenn die Streben radial zu der Vorrichtung (14) gedehnt werden.

2. Verfahren nach Anspruch 1, wobei die Schicht die Form eines Rohrs aufweist.

3. Verfahren nach Anspruch 1, wobei die Schicht eine vorbestimmte Dicke T aufweist, und die Streben jeweils eine vorbestimmte Breite W aufweisen, wobei das Verhältnis T:W in dem Bereich von 1:5 bis 1:9 liegt.

4. Selbst ausdehnbare dreidimensionale Vorrichtung (14), wobei die Vorrichtung ein Muster aufweist, welches mehrere Streben (13) umfasst, wobei deren gegenüberliegenden Enden jeweils mit gegenüberliegenden Enden von benachbarten Streben (13) verbunden sind und wobei die Streben (13) Mittelabschnitte zwischen ihren gegenüberliegenden Enden mit Öffnungen (15) zwischen den Mittelabschnitten von benachbarten Streben (13) aufweisen, wobei das Muster darüber hinaus die Öffnungen (15) umfasst, welche eine Länge entlang einer Achse der Vorrichtung (14) und eine Breite orthogonal zu der Achse aufweisen, wobei die Länge um einen Betrag länger als die Breite ist, welcher ausreicht, damit sich die Streben (13) in einer radialen Richtung orthogonal zu der Achse ausdehnen können, wenn die Streben (13) von einer langen schmalen Öffnungsform zu einer offenen Rautenform gedehnt werden, **dadurch gekennzeichnet, dass** die Vorrichtung (14) eine dünnschichtige Vorrichtung (14) ist, welche eine dünnschichtige Vorrichtungsschicht aus einer Formgedächtnis-Legierung umfasst, und dass sich einige der Streben (13) aus der Ebene biegen und einige auf der Ebene verbleiben, wenn sie radial gedehnt werden.

5. Vorrichtung (14) nach Anspruch 4, wobei die Schicht die Form eines Rohrs aufweist.

6. Vorrichtung (14) nach Anspruch 4, wobei die Schicht eine vorbestimmte Dicke T aufweist und die Streben (13) jeweils eine vorbestimmte Breite W aufweisen, wobei das Verhältnis T:W in dem Bereich von 1:5 bis 1:9 liegt.

7. Vorrichtung (14) nach Anspruch 4 und darüber hinaus einen inneren Stent (21) umfassend, und wobei die Schicht die Form eines hohlen Rohrs (19) aufweist, welches die Außenseite des inneren Stents (21) umschließt.

## Revendications

1. Procédé pour fabriquer un dispositif tridimensionnel autodéployable (14), comportant les étapes de formation d'une couche de matière du dispositif et de modelage de la couche du dispositif sous la forme d'un motif comprenant une pluralité d'entretoises (13) dont chacune a ses extrémités opposées reliées à des extrémités opposées d'entretoises adjacentes (13), et les entretoises (13) ayant des parties médianes entre leurs extrémités opposées avec des ouvertures (15) entre les parties médianes d'entretoises adjacentes (13), le motif étant en outre modelé de façon que les ouvertures (15) aient une longueur dans le sens longitudinal d'un premier axe du dispositif (14) et une largeur orthogonale au premier axe, la longueur étant plus grande que la largeur dans une mesure suffisante pour permettre aux entretoises (13) de se déployer dans une direction radiale orthogonale au premier axe lorsque les entretoises (13) s'étirent, passant d'une forme longue et étroite des ouvertures à une forme ouverte en losange ; **caractérisé en ce que** : la couche de matière du dispositif est une couche de film mince du dispositif, comprenant un alliage à mémoire de forme, si bien que le dispositif (14) est un dispositif à couche mince, et **en ce que**, lorsque les entretoises s'étirent dans la direction radiale du dispositif (14), certaines entretoises (13) s'incurvent hors du plan et certaines restent dans le plan.

2. Procédé selon la revendication 1, dans lequel la couche à la forme d'un tube.

3. Procédé selon la revendication 1, dans lequel la couche a une épaisseur donnée T et les entretoises ont chacune une largeur donnée W, le rapport T/W étant de 1/5 à 1/9.

4. Dispositif tridimensionnel autodéployable (14), le dispositif ayant un motif comprenant une pluralité d'entretoises (13) dont chacune a ses extrémités opposées reliées à des extrémités opposées d'entretoises adjacentes (13), et les entretoises (13) ayant des parties médianes entre leurs extrémités opposées avec des ouvertures (15) entre les parties médianes d'entretoises adjacentes (13), le motif comprenant en outre les ouvertures (15) qui ont une longueur dans le sens longitudinal d'un premier axe du dispositif (14) et une largeur orthogonale au premier axe, la longueur étant plus grande que la largeur dans une mesure suffisante pour permettre aux entretoises (13) de se déployer dans une direction radiale orthogonale au premier axe lorsque les entretoises (13) s'étirent, passant d'une forme longue et étroite des ouvertures à une forme ouverte en losange ; **caractérisé en ce que** : le dispositif (14) est un dispositif (14) comprenant une couche de film mince du dispositif, en alliage à mémoire de forme, et **en ce que** certaines entretoises (13) s'incurvent hors du plan et certaines restent dans le plan lorsqu'elles s'étirent dans la direction radiale.

5. Dispositif (14) selon la revendication 4, dans lequel la couche a la forme d'un tube.

6. Dispositif (14) selon la revendication 4, dans lequel la couche a une épaisseur donnée T et les entretoises (13) ont chacune une largeur donnée W, le rapport T/W étant de 1/5 à 1/9.

7. Dispositif (14) selon la revendication 4 et comportant en outre un stent intérieur (21), et la couche a la forme d'un tube creux (19) qui englobe l'extérieur du stent intérieur (21).
